# EUROPEAN PATENT SPECIFICATION

(11) **EP 2 713 114 B1**
(45) Date of publication and mention of the grant of the patent: **04.11.2020**
(21) Application number: 13186343.3
(22) Date of filing: 27.09.2013
(51) Int. Cl.: F24F 3/16, F24F 1/00, F24F 1/0007, F24F 11/30, F24F 120/12, F24F 11/79, F24F 11/89, F24F 11/74

(54) **Air conditioner with ionizer**
Klimaanlage mit Ionisierer
Conditionneur d'air doté d'un ioniseur

(30) Priority: 28.09.2012 KR 20120109094
(43) Date of publication of application: 02.04.2014
(73) Proprietor: LG ELECTRONICS INC., Youngdungpo-gu Seoul 150-721 (KR)
(72) Inventor: Park, Dongryul, 137-724 Seoul (KR); Yoon, Kyungsoo, 137-724 Seoul (KR); Lee, Jangwoo, 137-724 Seoul (KR); Sung, Bongjo, 137-724 Seoul (KR); Gwak, Jongseong, 137-724 Seoul (KR)
(74) Representative: Vossius & Partner Patentanwälte Rechtsanwälte mbB

(56) References cited:
- WO-A1-2011/045979
- GB-A- 2 260 830
- JP-A- 2004 347 264
- JP-A- 2005 114 195

## Description

The present invention relates to an air conditioner with an ionizer, and more specifically the present invention relates to an air conditioner having a movable ionizer arranged in the vicinity of an air outlet.

As environmental pollution becomes the worse, people having different respiratory disease or showing allergy reaction caused by polluted air increases the more. Consequently, various attempts, such as generation of anions, have been made for cleaning the polluted air to improve a quality of the air.

Since the anion, a molecule of oxygen, nitrogen, or so on having a negative charge, is very beneficial for a human body, and effective for removal of dust and odor, an ionizer module is additionally mounted to, and used in, different types of domestic appliances, such as an air conditioner, or air cleaner.

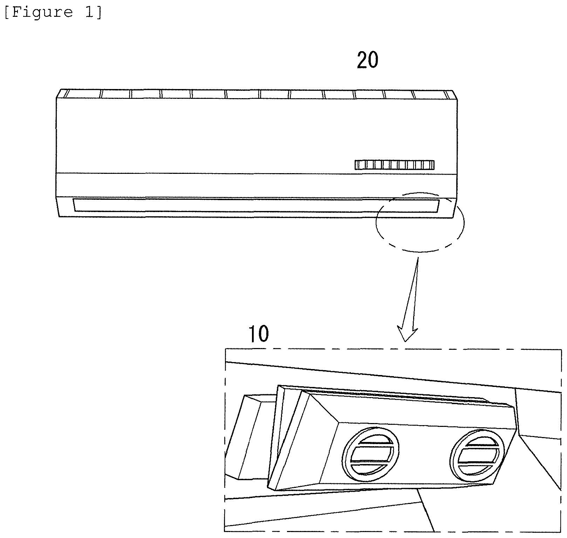

Referring to FIG. 1, as an example, mounted in the vicinity of an air outlet of a wall mounting type air conditioner 20 additionally, there may a small sized modular ionizer 10 for generating at least one of anion and cation.

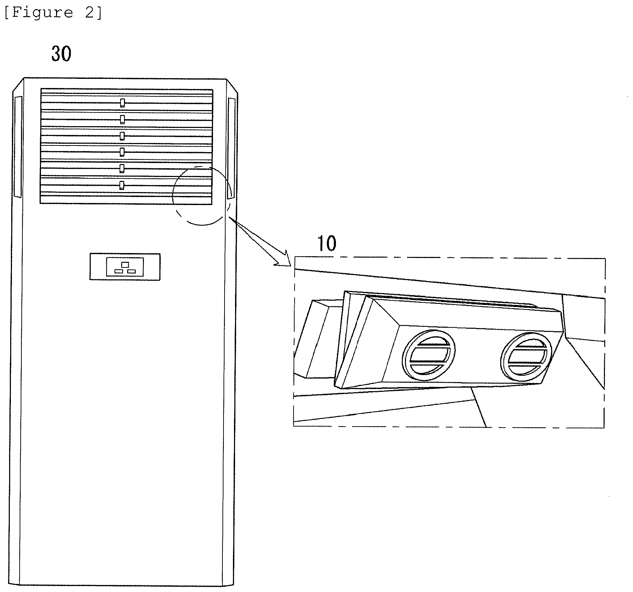

Referring to FIG. 2, the ionizer 10 may be mounted to a stand type air conditioner 30 in the vicinity of an air outlet thereof in a small sized modular mode for generating at least one of the anion and the cation.

However, since the ionizer 10 is mounted secured to a side, i.e., a left side or a right side, of the air outlet of the domestic appliance, such as the air cleaner or the air conditioner due to limitation of a mounting space, diffusion of the ions carried by air being discharged is limited, and, moreover, since the ions are discharged in a direction, not relevant to a position of a user, the ionizer 10 has a problem in that the user fails to feel an effect of the anion, properly. JP 2005 114195 A relates to an air conditioner according to the preamble of claim 1 including an ion generating device which is capable of uniformly distributing ions in a room, especially in the corners.

Accordingly, the present invention has been made in an effort to solve the aforementioned problems, and it is an object of the present invention to provide an air conditioner which makes ions generated by an ionizer to diffuse effectively owing to an air flow.

It is another object of the present invention to provide an air conditioner which makes ions to be forwarded to a person in a room, effectively.

The present invention provides an air conditioner according to claim 1 including, among other features, an ionizer to generate ions mounted on a vicinity of an air outlet, a carrier to move the ionizer, and a controller to control the ionizer and the carrier.

In accordance with an embodiment of the present invention, the air conditioner may further comprise a front chassis, and the ionizer is mounted on an inside of the front chassis.

In accordance with an embodiment of the present invention, the carrier includes a step motor and a lead screw, and the ionizer may move interlocked with the lead screw when the motor rotates the lead screw.

In accordance with an embodiment of the present invention, the step motor and the lead screw may be mounted on an inside of the air outlet.

In accordance with an embodiment of the present invention, the controller may control the carrier to move the ionizer to an initial position when power is supplied to the air conditioner or the power is changed from turn off to turn on.

In accordance with an embodiment of the present invention, the controller may control a position of the ionizer with a number of pulses applied to the step motor from the initial position.

In accordance with an embodiment of the present invention, the air conditioner further includes a sensor to sense a position of a person, and the controller may control the position of the ionizer through the carrier in response to a sensing signal of the sensor.

In accordance with an embodiment of the present invention, the sensor may be one of an ultrasonic sensor, an infrared sensor, and a heat sensor.

In accordance with an embodiment of the present invention, the sensor may be mounted to a front of the air conditioner.

In accordance with an embodiment of the present invention, the controller may determine a range a person presents with respect to the air conditioner with the sensor, and may control the carrier to make the ionizer to reciprocate within the range determined by the controller.

In accordance with an embodiment of the present invention, if the controller sets the ionizer to reciprocate, the controller may control the ionizer to increase an ion generation rate when the ionizer passes the range the person presents with respect to the air conditioner.

In accordance with an embodiment of the present invention, if the controller sets the ionizer to reciprocate, the controller may increase strength of air flow from the air conditioner when the ionizer passes the range the person presents with respect to the air conditioner.

In accordance with an embodiment of the present invention, the air conditioner further comprises an input unit, wherein an operation mode among a plurality of operation modes are set through the input unit.

In accordance with an embodiment of the present invention, the operation mode may be a water fall mode in which the controller controls an operation of the ionizer to set an ion generation rate to a highest, as well as move a position of the ionizer to a center of the air conditioner, to diffuse the ions at the center, and the controller sets an air flow rate of the air conditioner to be higher than other modes among the plurality of modes.

In accordance with an embodiment of the present invention, the operation mode may be a sleeping mode in which the controller controls an operation of the ionizer to set an ion generation rate to a lowest, as well as move a position of the ionizer to a left side and a right side of the air conditioner alternately at predetermined time intervals, to spread the generated ions.

In accordance with an embodiment of the present invention, the air conditioner further includes a vane driver to drive a vane which controls a direction of air being discharged, and the controller may control the carrier to reciprocate the ionizer periodically, controls the vane driver to swing the vane periodically, and synchronizes a reciprocating period of the ionizer and a swing period of the vane at a predetermined ratio to distribute ion concentration uniformly.

In accordance with an embodiment of the present invention, the air conditioner further includes a vane driver to drive the vane which controls a direction of the air being discharged, and the controller may move the position of the ionizer taking a position the person presents with respect to the air conditioner determined by the controller with the sensor and the controller may take into account direction of the vane in a state the vane is set to swing.

In accordance with an embodiment of the present invention, when the vane rotates to direct the air to a right side, the controller may control the carrier to position the ionizer at a left side, and, when the vane rotates to direct the air to the left side, the controller may control the carrier to position the ionizer at the right side.

### BRIEF DESCRIPTION OF THE DRAWINGS

FIG. 1 is a front view illustrating a general wall mounting type air conditioner having an ionizer module mounted thereto;
FIG. 2 is a front view illustrating a general stand type air conditioner having an ionizer module mounted thereto;
FIG. 3 is a perspective view illustrating an ionizer applicable to the present invention;
FIG. 4 is a front view illustrating an ionizer movable in a horizontal direction in an air conditioner in accordance with an embodiment of the present invention;
FIGS. 5A and 5B are schematic views illustrating an ionizer mounted to an inside structure in the vicinity of an air outlet in an air conditioner in accordance with an embodiment of the present invention;
FIG. 6 is a block diagram of an air conditioner in accordance with an embodiment of the present invention;
FIG. 7 is a schematic view of an ionizer in an air conditioner in accordance with an embodiment of the present invention illustrating the ionizer moving toward a user.

### DETAILED DESCRIPTION OF THE INVENTION

In what follows, an ionizer according to preferred embodiments of the present invention will be described in detail with reference to the appended drawings.

The air conditioner in accordance with an embodiment of the present invention may have a movable ionizer mounted in the vicinity of an air outlet. For an example, the air conditioner of the present invention may include an ionizer for generating ions, and a carrier for moving the ionizer. In the specification, the air conditioner is a machine for controlling a temperature, humidity, and an air quality of a space having an air cleaner, an air heater, an air cooler, a humidifier, a fan, and so on mounted therein.

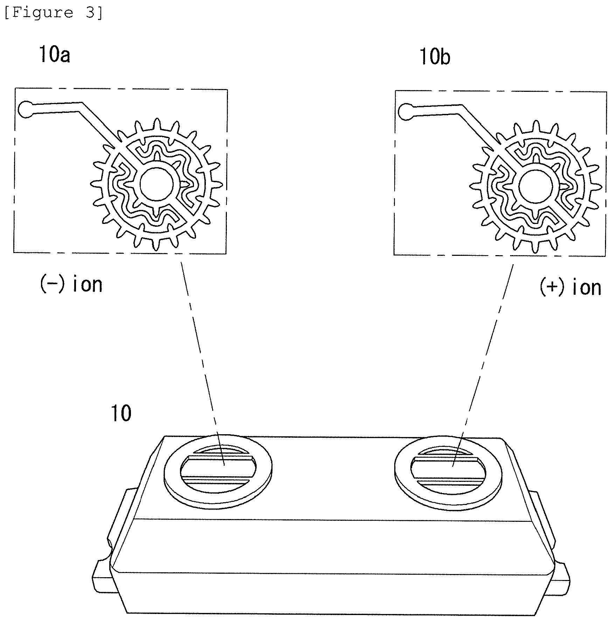

Referring to FIG. 3, the ionizer 10 may be in a small sized modular mode having an anion electrode 10a for generating an anion, and a cation electrode 10b for generating a cation.

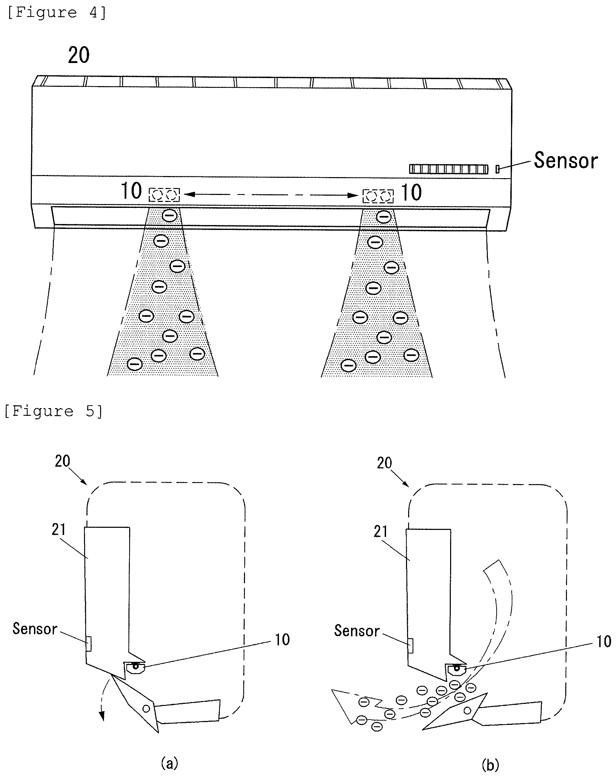

And, referring to FIG. 4, the modular ionizer 10 may be mounted in the vicinity of an outlet extended in a horizontal direction of the air conditioner, such that the modular ionizer 10 is movable along the air outlet in response to a signal, applied by a user, or from a human body sensor.

And, referring to FIG. 5, if the ionizer 10 is mounted to, for an example, to a wall mounting type air conditioner 20, the ionizer 10 may be mounted to a structure on an inside of a front chassis 21 for making the ionizer 10 invisible from an outside of the air conditioner 20, and making the anions to be discharged following an air flow of the air conditioner being discharged through the outlet.

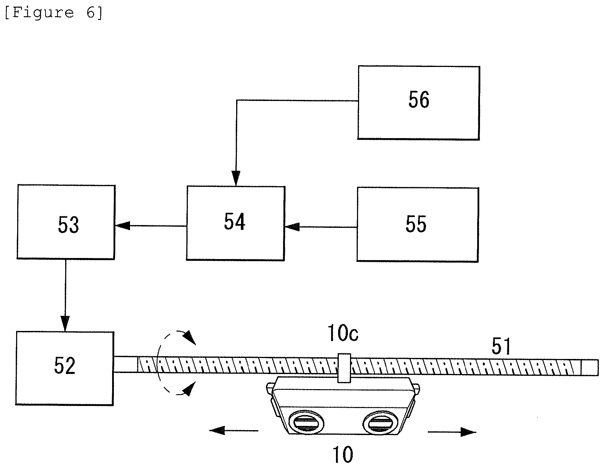

Referring to FIG. 6, the ionizer 10 moves, for an example, along a rail 51 mounted in a horizontal direction, wherein the rail 51 may be a lead screw rotated by driving the motor 52.

And, referring to FIG. 6, since the lead screw has a helical groove (A thread) formed therein, and the ionizer 10 has link ring 10c with a needle or a rack formed therein to be engaged with the groove (Thread) in the lead screw, if the lead screw rotates, the ionizer 10 moves in a length direction of the rail.

The motor 52 may be, for an example, a step motor for controlling rotation of a rotor in steps to control a position of a component connected to the lead screw accurately, wherein, as shown in FIG. 6, a driving unit 53 which applies a voltage to the motor 52 is operated under the control of a controller 54, such as a microcomputer. The position of the ionizer 10 may be controlled with a number of pulses, i.e., a number of steps, applied to the step motor in a feed forward method, without using an additional position sensor.

And, the controller 54 may move the position of the ionizer 10 to any position by controlling operation of the driving unit 53 in response to a signal applied by the user received through an input unit 55, or may move the position of the ionizer 10 to a position in the vicinity of a direction in which a human body is sensed by controlling operation of the driving unit 53 in response to a body sensed signal sensed by a sensor unit 56.

For an example, the input unit 55 may include a remote controller receiver module or a key panel for making the driving unit 53 to perform an operation the user desires selectively, and the sensor unit 56 may include at least one of different types of human body sensors, such as an ultrasonic sensor, an infrared sensor, a heat sensor, and so on for automatic sensing of the position of the user.

And, referring to FIG. 5, the human body sensor may be mounted to the front chassis 21 of the air conditioner, for an example, to a front of the air outlet for sensing the position of the user.

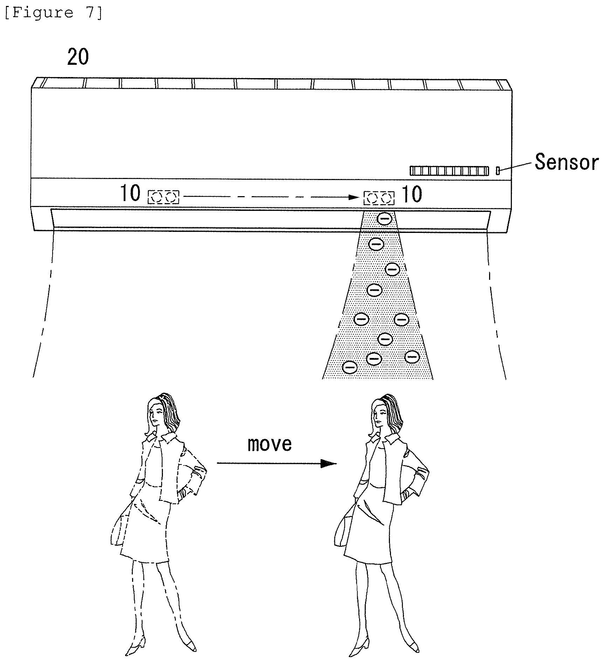

Referring to FIG. 7, upon reception of, for an example, the body sensed signal from the sensor unit 56, the controller 54 controls operation of the driving unit 53 in response to the human body sensed signal, to make a position of the ionizer 10 to direct the user.

For an example, if the user moves from a left side of the air conditioner to a right side thereof, the sensor unit 56 outputs the human body sensed signal corresponding to the movement of the user, and the controller 54 controls the operation of the driving unit 53, to control to vary the voltage to be applied to the motor 53.

According to this, the position of the ionizer 10 is moved from the left side to the right side along the rail 51 which rotates as the motor 53 is driven, making the ions to emit from the ionizer 10 toward the user moved to the right side, an ion effect the user feels can be improved, effectively.

And, if the signal applied by the user received through the input unit 55 is the remote controller signal corresponding to the position control on the ionizer, the controller 54 controls operation of the driving unit 53 to make the position of the ionizer 10 to move in response to the signal applied by the user.

The controller 54 sets a specific operation mode according to the signal applied by the user received through the input unit 55, wherein the specific operation mode may be any one of a plurality of operation modes set in advance for changing an ion generation rate, an air flow rate, or the like.

For example, if the specific mode is a water fall mode in which the ion generation rate is set to a highest, the controller 54 controls the operation of the ionizer 10 to increase the ion generation rate to a highest, as well as move the position of the ionizer 10 to a center, to diffuse the ions at the center intensively. In this time, the air flow rate of the air conditioner may be increased compared to a prior operation mode.

Opposite to this, if the specific mode is, for an example, a sleeping mode in which the ion generation rate is set to a lowest, the controller 54 controls the operation of the ionizer 10 to reduce the ion generation rate to a lowest as well as make the position of the ionizer 10 to move to the left side and the right side alternately at predetermined time intervals, to spread the ions generated thus to the left side and the right side widely while the user sleeps.

And, if a system power of the air conditioner is turned off, or an initializing operation is performed in response to the signal applied by the user, the controller 54 controls the operation of the driving unit 53 to move the position of the ionizer 10 to an initial point set in advance.

For an example, the controller 54 may make precise control on the step motor by moving the ionizer 10 to the closest one of the initial points and initializing a value of the step count counted up to now.

In order to make the controller 54 to be able to determine and control the position of the ionizer 10 even after supply of the system power fails suddenly during operation of the air conditioner, the controller 54 may control the driving unit 53 to move the ionizer 10 to one of the initial points when the power is supplied to the air conditioner or the power is changing from turn off to turn on. The controller 54 can determine the position of the ionizer 10 with the number of pulses, i.e., the number of steps, applied to the step motor through the driving unit 53 from the initial point.

In the meantime, the air conditioner may include a vane for controlling a direction of the air being discharged, and a vane driver for driving the vane, and, in order to control a distribution of ion concentration of a room, the controller 54 may control the position of the ionizer 10 interlocked with the direction of the vane, or strength of the air being discharged by the air conditioner or the ion generation rate of the ionizer 10 interlocked with the position of the ionizer 10.

If it is intended to distribute the concentration of the ions in the room uniformly, the controller 54 may control the driving unit 53 to reciprocate the ionizer 10 periodically, and, additionally, to make the vane of the air conditioner to swing, to distribute the ions generated by the ionizer far and uniformly, wherein, by synchronizing a reciprocating period of the ionizer 10 and a swing period of the vane, in an one to one ratio, in an integer ratio, or in a rational number ratio, it may also be possible to make the vane to have angles different from one another every time the ionizer 10 passes respective positions within a reciprocating section.

The controller 54 can grasp a range the user presents thereto from the human body sensed signal detected by the sensor unit 56 and control the driving unit 53 to make the ionizer 10 to reciprocate within the range grasped thus, or, in a state the ionizer 10 is set to reciprocate, control the ionizer 10 to increase the ion generation rate when the ionizer 10 passes the range, or control a fan (Not shown) which generates an air flow to increase a strength of the air flow or a flow rate of the air.

Moreover, the controller 54 can also move the position of the ionizer 10 taking the position of the user and the direction of the vane into account in a state the vane which controls a direction of the air being discharged from the air conditioner is set to swing, wherein it may be possible that the ions generated by the ionizer 10 can be focused on the user according to the air flow of which direction is controlled by the vane by making the ionizer 10 to position at a left side further than the user, if the vane rotates to direct the air being discharged to the right side, and by making the ionizer 10 to position at the right side further than the user, if the vane rotates to direct the air being discharged to the left side.

As has been described, the air conditioner with an ionizer of the present invention has the following advantage.

A range of ion diffusion can be controlled more efficiently, an ion effect the user feels can be improved by making to discharge the ions to a direction the user presents thereto.

While this invention has been described in connection with what is presently considered to be practical exemplary embodiments, it is to be understood that technical aspects of the present invention are not limited to the exemplary embodiments suggested in the specification, but, though a person of an ordinary skill in this field of art who understand the technical aspects of the present invention can suggest another exemplary embodiment by modifications, changes, removal, and addtion of constituent elements within a range of technical aspects the same with the present invention, it may also be within the scope of the present invention which is defined by the appended claims.

## Claims

1. An air conditioner (20) comprising:
an ionizer (10) to generate ions mounted on a vicinity of an air outlet;
a carrier (51, 52) to move the ionizer;
a vane driver (53) to drive a vane which controls a direction of air being discharged;
an input unit (55) to set an operation mode among a plurality of operation modes; and
a controller (54) to control a position of the ionizer through the carrier,
**characterized in that**
the air conditioner further comprises a sensor (56) to sense a position of a person; and
the controller is configured to control the position of the ionizer interlocked with a direction of the vane, strength of the air being discharged by the air conditioner, or an ion generation rate of the ionizer, or is configured to control the position of the ionizer according to a mode select signal inputted through the input unit.

2. The air conditioner as claimed in claim 1, wherein the air conditioner further comprises a front chassis (21), and the ionizer is mounted on an inside of the front chassis.

3. The air conditioner as claimed in claim 1 or 2, wherein the carrier includes a step motor (52) and a lead screw (51), and the ionizer moves interlocked with the lead screw when the motor rotates the lead screw.

4. The air conditioner as claimed in claim 3, wherein the step motor and the lead screw are mounted on an inside of the air outlet.

5. The air conditioner as claimed in claim 3 or 4, wherein the controller controls the step motor to move the ionizer to an initial position when power is supplied to the air conditioner or the power is changed from turn off to turn on.

6. The air conditioner as claimed in claim 5, wherein the controller is configured to control a position of the ionizer with a number of pulses applied to the step motor from the initial position.

7. The air conditioner as claimed in one of the preceding claims, wherein the sensor is one of an ultrasonic sensor, an infrared sensor, and a heat sensor.

8. The air conditioner as claimed in claim 7, wherein the sensor is mounted to a front of the air conditioner.

9. The air conditioner as claimed in any one of claims 7 or 8 wherein the controller is configured to determine a range a person presents with respect to the air conditioner with the sensor, and to control the carrier to make the ionizer to reciprocate within the range determined by the controller.

10. The air conditioner as claimed in claim 9, wherein, if the controller sets the ionizer to reciprocate, the controller is configured to control the ionizer to increase an ion generation rate when the ionizer passes the range the person presents with respect to the air conditioner.

11. The air conditioner as claimed in claim 9, wherein, if the controller sets the ionizer to reciprocate, the controller is configured to increase strength of air flow from the air conditioner when the ionizer passes the range the person presents with respect to the air conditioner.

12. The air conditioner as claimed in any one of claims 7 to 11,
wherein the controller controls the carrier to reciprocate the ionizer periodically, controls the vane driver to swing the vane periodically, and synchronizes a reciprocating period of the ionizer and a swing period of the vane at a predetermined ratio to distribute ion concentration uniformly.

13. The air conditioner as claimed in any one of claims 7 to 11,
wherein the controller moves the position of the ionizer taking a position the person presents with respect to the air conditioner determined by the controller with the sensor and the controller takes into account direction of the vane in a state the vane is set to swing.

14. The air conditioner as claimed in claim 13, wherein, when the vane rotates to direct the air to a right side, the controller controls the carrier to position the ionizer at a left side, and, when the vane rotates to direct the air to the left side, the controller controls the carrier to position the ionizer at the right side.

15. The air conditioner as claimed in one of the preceding claims, wherein the operation mode includes a water fall mode and a sleeping mode.

## Patentansprüche

1. Klimaanlage (20) aufweisend:
einen in einer Nähe eines Luftauslasses angebrachten Ionisierer (10) zur Erzeugung von Ionen;
einen Träger (51, 52) zum Bewegen des Ionisierers;
einen Flügelantrieb (53) zum Antreiben eines Flügels, der eine Richtung, in der Luft ausgestoßen wird, steuert;
eine Eingabeeinheit (55) zum Einstellen eines Betriebsmodus von mehreren Betriebsmodi; und
eine Steuereinrichtung (54) zur Steuerung einer Position der Ionisierers mit Hilfe des Trägers,
**dadurch gekennzeichnet, dass** die Klimaanlage ferner einen Sensor (56) zum Detektieren einer Position einer Person aufweist, und die Steuerungseinrichtung konfiguriert ist, die Position des Ionisierers in Zusammenwirken mit einer Richtung des Flügels, einer Stärke der von der Klimaanlage ausgestoßenen Luft oder einer Ionenerzeugungsrate des Ionisierers zu steuern, oder konfiguriert ist, die Position des Ionisierers gemäß eines über die Eingabeeinheit eingegebenen Modusauswahlsignals zu steuern.

2. Klimaanlage nach Anspruch 1, wobei die Klimaanlage ferner ein vorderes Gehäuse (21) aufweist und der Ionisierer an einer Innenseite des vorderen Gehäuses angebracht ist.

3. Klimaanlage nach Anspruch 1 oder 2, wobei der Träger einen Schrittmotor (52) und eine Führungsschraube (51) aufweist und der Ionisierer sich in Zusammenwirken mit der Führungsschraube bewegt, wenn der Motor die Führungsschraube rotiert.

4. Klimaanlage nach Anspruch 3, wobei der Schrittmotor und die Führungsschraube an einer Innenseite des Luftauslasses angebracht sind.

5. Klimaanlage nach Anspruch 3 oder 4, wobei die Steuereinrichtung den Schrittmotor steuert, um den Ionisierer in eine Anfangsposition zu bewegen, wenn die Klimaanlage mit Energie versorgt wird oder die Energie von Aus zu Ein geschaltet wird.

6. Klimaanlage nach Anspruch 5, wobei die Steuereinrichtung konfiguriert ist, eine Position des Ionisierers mit einer Anzahl von Pulsen, die von der Anfangsposition aus an den Schrittmotor angelegt werden, zu steuern.

7. Klimaanlage nach einem der vorstehenden Ansprüche, wobei der Sensor ein Ultraschallsensor oder ein Infrarotsensor oder ein Wärmesensor ist.

8. Klimaanlage nach Anspruch 7, wobei der Sensor an einer Vorderseite der Klimaanlage angebracht ist.

9. Klimaanlage nach einem der Ansprüche 7 oder 8, wobei die Steuereinrichtung konfiguriert ist, einen Bereich, den eine Person bezüglich der Klimaanlage hat, mit Hilfe des Sensors zu bestimmen, und den Träger zu steuern, um den Ionisierer in dem von der Steuereinrichtung bestimmten Bereich hin und her zu bewegen.

10. Klimaanlage nach Anspruch 9, wobei, wenn die Steuereinrichtung den Ionisierer hin und her bewegen lässt, die Steuereinrichtung konfiguriert ist, den Ionisierer zu steuern, um eine Ionenerzeugungsrate zu erhöhen, wenn der Ionisierer sich an dem Bereich, den die Person bezüglich der Klimaanlage hat, vorbei bewegt.

11. Klimaanlage nach Anspruch 9, wobei, wenn die Steuereinrichtung den Ionisierer hin und her bewegen lässt, die Steuereinrichtung konfiguriert ist, die Stärke des Luftstroms aus der Klimaanlage zu erhöhen, wenn der Ionisierer sich an dem Bereich, den die Person bezüglich der Klimaanlage hat, vorbei bewegt.

12. Klimaanlage nach einem der Ansprüche 7 bis 11, wobei die Steuereinrichtung den Träger steuert, um den Ionisierer periodisch hin und her zu bewegen, den Flügelantrieb steuert, um den Flügel periodisch zu schwingen, und eine Hin-und-Her-Bewegung-Periode des Ionisierers und eine Schwingungsperiode des Flügels mit einem vorgegebenen Verhältnis synchronisiert, um eine Ionenkonzentration gleichmäßig zu verteilen.

13. Klimaanlage nach einem der Ansprüche 7 bis 11,
wobei die Steuereinrichtung die Position des Ionisierers bewegt, unter Einnahme einer von der Steuereinrichtung mit Hilfe des Sensors bestimmten Position, die die Person bezüglich der Klimaanlage hat, und in einem Zustand, wenn der Flügel zum Schwingen eingestellt ist, die Steuereinrichtung eine Richtung des Flügels berücksichtigt.

14. Klimaanlage nach Anspruch 13, wobei, wenn der Flügel rotiert, um die Luft zu einer rechten Seite zu lenken, die Steuereinrichtung den Träger steuert, um den Ionisierer an einer linken Seite zu positionieren, und wenn der Flügel rotiert, um die Luft zu der linken Seite zu lenken, die Steuereinrichtung den Träger steuert, um den Ionisierer an der rechten Seite zu positionieren.

15. Klimaanlage nach einem der vorstehenden Ansprüche, wobei der Betriebsmodus einen Wasserfallmodus und einen Ruhemodus aufweist.

## Revendications

1. Climatiseur (20) comprenant :
un ioniseur (10) pour générer des ions monté au voisinage d'une sortie d'air ;
un support (51, 52) pour déplacer l'ioniseur ;
un élément d'entraînement d'ailette (53) pour entraîner une ailette qui commande une direction d'air évacué ;
une unité d'entrée (55) pour régler un mode de fonctionnement parmi une pluralité de modes de fonctionnement ; et
un dispositif de commande (54) pour commander une position de l'ioniseur à travers le support,
**caractérisé en ce que**
le climatiseur comprend en outre un détecteur (56) pour détecter une position d'une personne ; et
le dispositif de commande est configuré pour commander la position de l'ioniseur inter-verrouillée avec une direction de l'ailette, une puissance de l'air évacué par le climatiseur, ou un taux de génération d'ions de l'ioniseur, ou est configuré pour commander la position de l'ioniseur selon un signal de sélection de mode entré via l'unité d'entrée.

2. Climatiseur selon la revendication 1, dans lequel le climatiseur comprend en outre un châssis avant (21), et l'ioniseur est monté sur un intérieur du châssis avant.

3. Climatiseur selon la revendication 1 ou 2, dans lequel le support comporte un moteur pas à pas (52) et une vis mère (51), et l'ioniseur se déplace de manière inter-verrouillée avec la vis mère lorsque le moteur fait tourner la vis mère.

4. Climatiseur selon la revendication 3, dans lequel le moteur pas à pas et la vis mère sont montés sur un intérieur de la sortie d'air.

5. Climatiseur selon la revendication 3 ou 4, dans lequel le dispositif de commande commande le moteur pas à pas pour déplacer l'ioniseur vers une position initiale lorsque l'alimentation est fournie au climatiseur ou que l'alimentation passe de la désactivation à l'activation.

6. Climatiseur selon la revendication 5, dans lequel le dispositif de commande est configuré pour commander une position de l'ioniseur avec un certain nombre d'impulsions appliquées au moteur pas à pas depuis la position initiale.

7. Climatiseur selon l'une quelconque des revendications précédentes, dans lequel le détecteur est l'un d'un détecteur à ultrasons, d'un détecteur infrarouge et d'un détecteur de chaleur.

8. Climatiseur selon la revendication 7, dans lequel le détecteur est monté à l'avant du climatiseur.

9. Climatiseur selon l'une quelconque des revendications 7 ou 8, dans lequel le dispositif de commande est configuré pour déterminer une plage que présente une personne par rapport au climatiseur par le détecteur et pour commander le support pour que l'ioniseur effectue un mouvement de va-et-vient dans la plage déterminée par le dispositif de commande.

10. Climatiseur selon la revendication 9, dans lequel,
si le dispositif de commande commande l'ioniseur pour effectuer un mouvement de va-et-vient, le dispositif de commande est configuré pour commander l'ioniseur pour augmenter un taux de génération d'ions lorsque l'ioniseur dépasse la plage que présente la personne par rapport au climatiseur.

11. Climatiseur selon la revendication 9, dans lequel, si le dispositif de commande commande l'ioniseur pour effectuer un mouvement de va-et-vient, le dispositif de commande est configuré pour augmenter la puissance du flux d'air provenant du climatiseur lorsque l'ioniseur dépasse la plage que présente la personne par rapport au climatiseur.

12. Climatiseur selon l'une quelconque des revendications 7 à 11,
dans lequel le dispositif de commande commande le support pour amener l'ioniseur à effectuer un mouvement de va-et-vient, commande le dispositif d'entraînement d'ailette pour faire osciller l'ailette périodiquement, et synchronise une période de va-et-vient de l'ioniseur et une période d'oscillation de l'ailette sur un rapport prédéterminé pour distribuer uniformément la concentration en ions.

13. Climatiseur selon l'une quelconque des revendications 7 à 11,
dans lequel le dispositif de commande déplace la position de l'ioniseur en prenant une position que la personne présente par rapport au climatiseur déterminée par le dispositif de commande avec le détecteur et le dispositif de commande prend en compte la direction de l'ailette dans un état dans lequel l'ailette est réglée pour osciller.

14. Climatiseur selon la revendication 13, dans lequel, lorsque l'ailette tourne pour diriger l'air vers un côté droit, le dispositif de commande commande le support pour positionner l'ioniseur sur un côté gauche, et, lorsque l'ailette tourne pour diriger l'air vers le côté gauche, le dispositif de commande commande le support pour positionner l'ioniseur sur le côté droit.

15. Climatiseur selon l'une quelconque des revendications précédentes, dans lequel le mode de fonctionnement comporte un mode cascade et un mode sommeil.
